# EUROPÄISCHE PATENTSCHRIFT

(11) **EP 0 484 472 B1**
(45) Veröffentlichungstag und Bekanntmachung des Hinweises auf die Patenterteilung: **16.07.1997**
(21) Anmeldenummer: 91906480.8
(22) Anmeldetag: 11.04.1991
(51) Int. Cl.: C12N 11/06

(54) **VERFAHREN ZUR LICHTINDUZIERTEN IMMOBILISIERUNG VON BIOMOLEKÜLEN AN CHEMISCH "INERTEN" OBERFLÄCHEN**
METHOD FOR THE LIGHT-INDUCED IMMOBILIZATION OF BIOMOLECULES ON CHEMICALLY "INERT" SURFACES
PROCEDE POUR L'IMMOBILISATION INDUITE PAR LA LUMIERE DE BIOMOLECULES SUR DES SURFACES CHIMIQUEMENT "INERTES"

(30) Priorität: 12.04.1990 CH 1253/90
(43) Veröffentlichungstag der Anmeldung: 13.05.1992
(73) Patentinhaber: SIGRIST, Hans, Dr., CH-2007 Neuchâtel (CH); KLINGLER-DABRAL, Vibhuti, D-64347 Griesheim (DE); DOLDER, Max, CH-3012 Bern (CH); WEGMUELLER, Bernhard, CH-3012 Bern (CH)
(72) Erfinder: SIGRIST, Hans, Dr., CH-2007 Neuchâtel (CH); KLINGLER-DABRAL, Vibhuti, D-64347 Griesheim (DE); DOLDER, Max, CH-3012 Bern (CH); WEGMUELLER, Bernhard, CH-3012 Bern (CH)
(86) Internationale Anmeldenummer: CH9100085
(87) Internationale Veröffentlichungsnummer: WO9116425

(56) Entgegenhaltungen:
- EP-A- 175 973
- US-A- 3 959 078
- US-A- 4 597 999
- JOURNAL OF LIPID RESEARCH, Band 25, 1984, New York, NY (US); C.A. LINGWOOD, Seiten 1010-1012
- PATENT ABSTRACTS OF JAPAN, Band 13, Nr. 174 (C-589)(3522), 25 April 1989
- BIOCHEMISTRY, Band 20, Nr. 24, Easton, PA (US); E.F. VANIN et al., Seiten 6754-6760

## Beschreibung

Die Erfindung betrifft ein Verfahren zur lichtinduzierten Immobilisierung von Biomolekülen in monomolekularen Schichten unter Verwendung von photoaktivierbaren Arylaziden oder Diazirinen als molekulare Klebstoffe. Ausgelöst durch die schnelle Entwicklung und Miniaturisierung von bioanalytischen Methoden einerseits, und den Fortschritten der Biosensortechnik andererseits besteht ein reges Interesse, die Wechselwirkung zwischen Biomolekülen und Oberflächen organisch-synthetischer oder anorganischer Natur besser zu verstehen. Gleichwertig in der Bedeutung ist die Entwicklung von Methoden zur wirksamen, chemisch stabilen Kopplung von Biomolekülen an Trägermaterialien, wobei erstere weder chemisch vorbehandelt noch extremen (Kopplungs)Reaktionsbedingungen ausgesetzt werden müssen. Analytisch/diagnostische Verfahren und die Herstellung von oberflächenaktiven Biosensoren erfordern eine geeignete Verankerung der Wirkstoffe in monomolekularen Schichten. Weil die Oberflächen vieler, zu diesem Zweck bisher eingesetzten Trägermaterialien keine oder wenig geeignete chemisch reaktive Funktionen besitzen, wurden Biomoleküle bisher meist oberflächendeckend mittels gruppenspezifischen, thermochemischen Reaktionen an vorbehandelte Trägermaterialien gebunden. Chemische Immobilisierungen dieser Art sind grundsätzlich möglich. Das Verfahren setzt jedoch das Vorhandensein von funktionellen Gruppen voraus, die sich gezielt aktivieren lassen. Zudem kann die monomolekulare Belegung von Oberflächen mit bisher verwendeten Verfahren lediglich oberflächendeckend durchgeführt werden (bulk-Verfahren).

Aus dem Stand der Technik sind ähnliche Verfahren bekannt; so beschreibt Lingwood [C.A. Lingwood, Journal of Lipid Research **25**, (1984) 1010] die Herstellung von Matrices mit einer gewissen Affinität bezüglich Glykolipiden unter Verwendung von heterobifunktionellen, quervernetzenden Agenzien. Dazu wird eine Technik eingesetzt, bei der ein photoaktivierbares, heterobifunktionelles Quervernetzungsreagens zur kovalenten Immobilisierung von Glykolipiden auf Agarose oder speziell derivatisierten Trägern aus Glas zur Anwendung kommen.

Als Trägermaterial kommt bei diesem Verfahren kein inerter Träger in Frage, sondern - neben Aminohexylagarose - durch Aminogruppen funktionalisierte Gläser, wie z.B. sog. Aminopropylgläser oder auch Aminophenylgläser.

Die weitere Derivatisierung der Aminomatrix erfolgt durch Vernetzen der Aminogruppen des entsprechend derivatisierten Glases entweder mit Hydroxysuccinimidylazidobenzoat (HSAB) oder mit Methyl-4-azidobenzimidat (MABI). Die zu bindenden Glycolipide werden - nach dem Waschen der Matrix - erst in einem zweiten Schritt in Lösung zugefügt und chemisch kovalent gebunden.

Daneben offenbart die US-Patentschrift 4 597 999 im allgemeinen Sinn eine Methode zur kovalenten Kupplung zweier molekularer Spezies. Insbesondere findet das in dieser Patentschrift beschriebene Verfahren Verwendung zur kovalenten Bindung Kohlenwasserstoffgruppen-aufweisender Liganden an eine entsprechende Matrix. - Auch das in diesem Dokument beschriebene Verfahren stellt an das Trägermaterial die Forderung, daß die Matrix freie Aminogruppen aufweist [Spalte 3, Zeile 21 ff.]. Als Beispiele sind u.a. sog. Aminophenyl- und Aminopropylgläser genannt.

Bei dem in der US-PS 4 597 999 beschriebenen Verfahren wird im ersten Schritt die aminogruppenhaltige Matrix mit einem heterobifunktionellen Quervernetzer - wie beispielsweise 4-Methylazidobenzimidat oder N-Hydroxysuccinimidylazidobenzoat umgesetzt, um die für dieses Verfahren benötigte aktivierte Matrix bereitzustellen. - In einem zweiten Reaktionsschritt erfolgt letztendlich die Kupplungsreaktion mit dem gewünschten Liganden [Spalte 4, Zeile 4ff.].

Des weiteren wird in der US-Patentschrift 3 959 078 ein Verfahren zur Enzymimmobilisierung beschrieben, bei dem Enzyme unter Anwendung eines thermochemischen und eines photochemischen Schritts immobilisiert werden.

Dabei wird in einem ersten Schritt ein bifunktionelles Angens, das im ursprünglichen Zustand einen thermochemisch aktivierbaren und einen photochemisch aktivierbaren Substituenten aufweist, in einen thermochemischen Schritt an eine geeignet derivatisierte Oberfläche eines Trägermaterials gebunden. In einem weiteren Schritt wird das so derivatisierte Trägermaterial auf photochemischem Wege aktiviert und das gewünschte Enzym somit im Rahmen eines photochemischen Schritts gebunden.

Zur Durchführung des Gesamtverfahrens ist somit grundlegende Voraussetzung, daß das Trägermaterial in geeigneter Form derivatisiert ist.

Während das bifunktionelle Agens im ersten thermochemischen Schritt in der Dunkelheit mit der Aminogruppe an der Oberfläche des Trägermaterials reagiert, erfolgt erst in einem zweiten photochemischen Schritt die eigentliche Immobilisierung des Enzyms.

Daneben offenbart die Europäische Offenlegungsschrift der Publikationsnummer 0 175 973 ein Trägermaterial zur Verwendung für die Immunbestimmung, bei dem ein Reaktionspartner der immunologischen Reaktion an das Trägermaterial über heterobifunktionelle photoaktivierbare Verbindungen, deren eine funktionelle Gruppe durch eine Acylacidgruppe gebildet wird, kovalent gebunden wird.

Wie dem einzigen Beispiel dieser Offenlegungsschrift entnommen werden kann, handelt es sich auch bei dem dort offenbarten Verfahren ebenfalls um ein mehrstufiges Verfahren, bei dem das Protein zunächst mit einem sog. "Brückenbildner" - wie zum Beispiel N-Succinimidyl-6-(4'-azido-2'-nitrophenylamino)hexan - an ein Trägermatrial gebunden wird. Nach den anschließenden Waschschritten wird das nunmehr trägergebundene Protein mit Lohmant's Reagenz umgesetzt.

Dieser zweite Brückenbildner reagiert mit dem N-Hydroxy-succinimidester als funktionelle Gruppe kovalent mit dem auf dem auf dem Träger gebundenen Protein. Nach erneuten Waschschritten kann das so derivatisierte trägergebundene Protein mit geeigneten Antikörper inkubiert werden, welche hierbei mit ihrem konstanten Teil an das Protein gebunden werden.

Des weiteren kann einem Referat im Patent Abstracts of Japan [Vol. 13, No. 174 (C-589)(3522) vom 25. April 1989] ein Verfahren entnommen werden, bei dem ein im wesentlichen wasser-unlöslicher Film aus einem organischen Material, der ein Precursor-Molekül zur Bildung eines Nitrens oder Carbens enthält zur Immobilisierung eingesetzt wird.

Dieser Film wird auf einem Substrat gebildet und in einem zweiten Schritt in Gegenwart eines bioaktiven Proteins mit Licht bestrahlt, was zu einer Immobilisierung des Proteins auf dem organischen Filmmaterial führt. Aus diesem Abstract geht somit hervor, daß zur Durchführung des Verfahrens ebenfalls eine zweistufige Vorgehensweise zwingend erforderlich ist.

Es ist die Aufgabe der vorliegenden Erfindung, makromolekulare Stoffe, insbesondere Biomoleküle, regiospezifisch und topologisch orientiert an chemisch "inerte" Oberflächen zu binden. Es sollen Methoden aufgezeigt werden, die erlauben, molekulare Schichten von biologisch aktiven Wirkstoffen (Eiweissmoleküle, Nukleinsäuren, Kohlehydrate, Lipide, niedermolekulare Wirkstoffe) über geeignete Vernetzer an feste Phasen (Trägermaterial) von unterschiedlicher chemischer Natur zu immobilisieren. Zur kovalenten Immobilisierung sollen photoaktivierbare Vernetzermoleküle, eingesetzt werden. Photoaktivierbare Reagenzien sind den thermo-chemischen Vernetzungsreaktionen überlegen, weil die Reaktion photo-optisch oder durch gezielte Applikation elektrischer Energie bezüglich Ort und Abmessung selektioniert und die Kopplungsreaktion zeitlich kontrolliert ausgelöst werden kann.

Diese Aufgabe wird durch das erfindungsgemäße Verfahren gelöst, das photochemische Immobilisierung von Biomolekülen an "inerten" Trägern in einem Schritt ermöglicht. Die kovalente Bindung der Biomoleküle an den Träger erfolgt über photogenerierte Carbene oder Nitrene. Carbene, ebenso wie Nitrene sind chemisch äusserst reaktive Zwischenprodukte. Sie sind geeignet, Biomoleküle durch Insertionsreaktionen in C-H, C-C, C=C, N-H, O-H, S-H Bindungen kovalent zu binden. Das resultierende breitgefächerte Reaktionsspektrum der photogenerierten Carbene und Nitrene übertrifft deshalb die thermo-chemischen Modifikationsreaktionen bezüglich den erforderten Reaktionsbedingungen. Durch Einsatz von Laserlichtquellen oder durch Applikation der, zur Aktivierung der reaktiven Funktionen erforderlichen Energie, können kleinste Oberflächen selektiv aktiviert und mit Biomolekülen monomolekular belegt werden.

Die photochemisch induzierte Immobilisierung von Liganden erfolgt unter Verwendung eines mehrfach derivierten Linkermoleküls in einem einzigen Reaktionsschritt.

### DAS ERFINDUNGSGEMÄßE, EINSTUFIGE KOPPLUNGSVERFAHREN BESTEHT AUS DEN FOLGENDEN TEILSCHRITTEN:

1. Ein Linkermolekül (z.B. ein synthetisches oder natürliches Polymer) wird mit heterobifunktionellen, photoaktivierbaren Vernetzermolekülen (z.B. 3-(Trifluoromethyl)-3-(m-isothiocyanophenyl)diazirin oder 3-(Trifluoromethyl)-3-(m-aminophenyl)diazirin, p-Azidophenylisothiocyanat oder p-Azidoanilin) mehrfach deriviert (= Photolinkerpolymer).
2. Das Photolinkerpolymer wird auf die "inerte" Oberfläche aufgetrocknet.
3. Zu immobilisierende Biomoleküle werden gelöst auf die photolinker-belegte Oberfläche aufgetragen. Das Lösungsmittel wird partiell oder gänzlich entfernt.
4. Durch Einstrahlen von Licht mit geeigneter Wellenlänge (Diazirine: 350 nm) werden die photoaktivierbaren funktionellen Gruppen aktiviert und die Kopplungsreaktion ausgelöst.
5. Nach erfolgter Photokopplung werden nicht-gebundene Liganden durch mehrmaliges Waschen der Oberfläche (z.B. durch Filtration) entfernt. Mit diesem Schritt können gleichzeitig Begleitsubstanzen (Pufferkomponenten, Salze, Detergentien) ausgetauscht oder aus dem System entfernt werden.

### BEISPIEL EINER ANWENDUNG DES EINSCHRITT-KOPPLUNGSVERFAHRENS

In Analogie zu bestehenden, auf Adsorption basierenden immunologischen Verfahren können Proteine, Peptide, Nukleinsäuren und Kohlehydrate in einem erstaunlich einfachen Prozess kovalent auf Mikrotiterplatten immobilisiert werden. Das Vorgehen verlangt keine spezielle Vorbehandlung des zu bindenden Biomoleküls und handelsübliche Trägermaterialien (z.B. Nunc Immunoplate Maxisorp) können ohne Vorbehandlung verwendet werden. Mikrotiterplatten werden mit einem Polymer (Polypeptid) belegt, welches vorgängig mehrfach mit photoaktivierbaren funktionellen Gruppen bestückt wurde (Photolinkerpeptid). Die Immobilisation erfolgt nach Belichtung durch Carben- insertion. Die am Trägermolekül angebrachten photoaktiven funktionellen Gruppen (z.B. Diazirine, reagieren gleichzeitig mit dem zu bindenden Liganden (z.B. Protein, DNS, Immunoglobulin) und mit der zu belegenden Oberfläche (z.B. Polystyrol).

### Herstellung des Photolinkerpeptides

Rinderserumalbumin (80 mg) wird in 14 ml TEA/HAc-Puffer, pH 10.5 (100 ml H₂O, 100 ml Aceton, 1 ml Triethylamin, 1 ml Essigsäure (2 M)) suspendiert und im Ultraschallbad beschallt bis die Lösung klar ist. Zu 24 µl 3-(Trifluoromethyl)-3-(m-aminophenyl)diazirin (TRIMID, hergestellt nach Dolder et al. (1990) J. Prot. Chem. 9, 407-415) in Tetrachlorkohlenstoff werden 6 ml Aceton gegeben. Proteinlösung und Reagens werden in einem 100 ml Rundkolben gemischt und während einer Stunde bei 70°C rückflussiert. Die Reaktionslösung wird anschliessend dreimal mit je 30 ml Heptan/Essigsäureethylester (6:3 v/v) extrahiert und die organische Phase verworfen. Die Wasserphase wird über Nacht lyophilisiert. Das trockene Produkt wird in 6 ml 0.4% (w/v) Natrium Dodecylsulfat in PBS (150 mM NaCl, 5 mM Natrium Phosphat pH 7,4) suspendiert und beschallt bis die Lösung klar ist. Zur weiteren Reinigung wird das Produkt an Sephadex G-15 medium in PBS chromatographiert und die vereinigten proteinhaltigen Fraktionen 48 Stunden gegen H₂O bidest dialysiert (Spectrapor cut off 6000-8000). Nach Lyophilisation wird das Produkt bei -20°C aufbewahrt.

### Belegen der "inerten" Oberfläche

Die Reaktionsgefässe von Titerplatten (z.B. Nunc-lmmuno Module, Polysorp F8) werden mit je 40 µl Photolinkerpeptid in H₂O (entsprechend 1 nMol TRIMID- deriviertem Rinderserumalbumin) versetzt. Der Boden des Reaktionsgefässes soll gleichmässig benetzt sein. Die Reaktionsgefässe werden anschliessend am Wasserstrahlvakuum während einer Stunde bei Raumtemperatur getrocknet. Derart belegte Titerplatten können lichtdicht verpackt bei -20°C mindestens 3 Monate aufbewahrt werden.

### Applikation der Biomoleküle und lichtinduzierte Immobilisierung

Die zur Immobilisierung eingesetzten Liganden werden in einem beliebigen Puffersystem gelöst (z.B. 1 mg Streptavidin in 2 ml 150 mM NaCl, 5 mM Natrium Phosphat Puffer, pH 7.4) und bis zur gewünschten Endkonzentration (z.B. 10 bis 1000 pMol Streptavidin pro 30 µl) verdünnt. Die mit Photolinkerpeptid belegten Reaktionsgefässe werden mit 30 µl Ligandlösung versetzt und zwei Stunden bei Raumtemperatur am Wasserstrahlvakuum getrocknet. Zur Photoaktivierung werden die Reaktionsgefässe 5 - 30 Minuten der Strahlung von UV Lichtquellen (z.B. parallel angeordnete UV (366 nm) Röhren, Silvania F8T5/BLB USA, 8 Watt) ausgesetzt, und anschliessend je 5 mal mit 150 mM NaCl, 5 mM Natrium Phosphat Puffer, pH 7.4, 2 mal mit H₂O und zweimal mit Alkohol gewaschen.

### Quantitativer Nachweis der Immobilisierung

Die Immobilisierung von Streptavidin wird durch Zugabe von radioaktiv markiertem [¹⁴C]-Biotin quantifiziert. Nach beschriebenem Verfahren immobilisierte Immunoglobuline können mit einem zweiten Antikörper komplexiert werden, welcher alkalische Phosphatase kovalent gebunden trägt und somit das Substrat p-Nitrophenylphosphat umsetzen kann. Freigesetztes p-Nitrophenol wird durch Absorptionsmessung (405 nm) in kommerziell erhältlichen ELISA-Reader Geräten quantitativ bestimmt. In analogem Vorgehen kann Digoxigenin markierte DNS gekoppelt und über anti-Digoxigenin Antikörper und alkalische Phosphatase nachgewiesen werden.

Die kovalente Immobilisierung von Biomolekülen verschiedener Klassen illustriert das breite Anwendungsspektrum und grosse Anwendungspotential des weitgehend standardisierten Verfahrens. Die Ausbeuten an gebundenen Molekülen sind gut. Das Verfahren lässt sich uneingeschränkt in bestehende Analyseprozesse (z.B. ELISA) integrieren. Nebst der Unabhängigkeit von funktionellen Gruppen am Liganden und der Unabhängigkeit von einschränkenden Reaktionsbedingungen, sind die Mehrfachverwendung Antigen-belegter Mikrotiterplatten und die einfach durchführbare Kopplung von Liganden von analytischer und verfahrenstechnischer Bedeutung.

Das Verfahren stellt erstmals eine anwendbare, erprobte Methode zur Immobilisierung von Biomolekülen dar, die bis zur Marktreife entwickelt werden konnte. Ein bedeutender Vorteil des beschriebenen Vorgehens ist die Tatsache, dass Diazirine bei fensterglas-gefiltertem Tageslicht gehandhabt werden können. Ihre Aktivierung erfolgt bei 350 nm mit kommerziell erhältlichen Beleuchtungsgeräten.

## Patentansprüche

1. Verfahren zur lichtinduzierten Immobilisierung von Biomolekülen, dadurch gekennzeichnet, daß die Biomoleküle auf photoaktivierbaren Trägermaterialien durch Einstrahlen von Licht an einer chemisch inerten Oberfläche in einer Stufe kovalent gebunden werden.

2. Verfahren nach Anspruch 1, dadurch gekennzeichnet, daß als Biomoleküle Eiweißmoleküle, Nukleinsäuren, Kohlehydrate Lipide oder niedermolekulare Wirkstoffe eingesetzt werden.

3. Verfahren nach Anspruch 1, dadurch gekennzeichnet, daß immunologisch aktive Moleküle, insbesondere Antikörper, Antigene oder Haptene unter Erhaltung ihrer biologischen Aktivität gebunden werden.

4. Verfahren nach Anspruch 1, dadurch gekennzeichnet, daß biologisch aktive Moleküle, insbesondere Enzyme oder Rezeptoren, zum Zweck der Herstellung von Biosensoren gebunden werden.

5. Verfahren nach Anspruch 1, dadurch gekennzeichnet, daß Eiweißmoleküle und/oder Kohlehydrate zur Vermeidung der Abstoßung körperfremder Substanzen photochemisch auf Oberflächen von Implantaten gebunden werden.

6. Verfahren nach Anspruch 1, dadurch gekennzeichnet, daß Biomoleküle, insbesondere Eiweißmoleküle oder Teile davon, zum Zweck der Herstellung von molekularen Schaltelementen gebunden werden.

## Claims

1. Process for the light-induced immobilisation of biomolecules, characterised in that the biomolecules on photoactivatable carrier materials are covalently bound in one step to a chemically inert surface by irradiation with light.

2. Process according to claim 1, characterised in that protein molecules, nucleic acids, carbohydrates, lipids or low-molecular weight active substances are used as the biomolecules.

3. Process according to claim 1, characterised by binding of immunologically active molecules, particularly antibodies, antigens or haptens, with retention of their biological activity.

4. Process according to claim 1, characterised by binding of biologically active molecules, particularly enzymes or receptors, in order to produce biosensors.

5. Process according to claim 1, characterised by photochemical binding of protein molecules and/or carbohydrates to surfaces of implants in order to prevent the rejection of substances foreign to the body.

6. Process according to claim 1, characterised by binding of biomolecules, particularly protein molecules or parts thereof, in order to produce molecular switching elements.

## Revendications

1. Procédé pour l'immobilisation induite par la lumière de biomolécules caractérisé en ce que les biomolécules sont fixées de manière covalente en une étape sur des matériaux supports photo-activables par projection de lumière sur une surface chimiquement inerte.

2. Procédé selon la revendication 1 caractérisé en ce que des molécules de protéines, des acides nucléiques, des glucides, des lipides ou des principes actifs de faible masse moléculaire sont utilisés comme biomolécules.

3. Procédé selon la revendication 1 caractérisé en ce que des molécules immunologiquement actives, en particulier des anticorps, des antigènes ou des haptènes, sont fixées avec conservation de leur activité biologique.

4. Procédé selon la revendication 1 caractérisé en ce que des molécules biologiquement actives, en particulier des enzymes ou des récepteurs, sont fixées en vue de la production de biocapteurs.

5. Procédé selon la revendication 1 caractérisé en ce que des molécules de protéines et/ou des glucides sont fixés par voie photochimique sur des surfaces d'implants pour éviter le rejet de substances étrangères à l'organisme.

6. Procédé selon la revendication 1 caractérisé en ce que des biomolécules, en particulier des molécules de protéines ou des parties de celles-ci, sont fixées en vue de la production d'éléments de circuits moléculaires.
